# EUROPEAN PATENT APPLICATION

(11) **EP 4 039 285 A1**
(43) Date of publication of application: **10.08.2022**
(21) Application number: 21155217.9
(22) Date of filing: 04.02.2021
(51) Int. Cl.: A61L 15/26, A61L 27/52, C08F 2/01, C08F 2/38, C08J 3/075

(54) **CONTROLLING THE TRIBOLOGICAL PROPERTIES OF GELS**

(71) Applicant: ETH Zurich, 8092 Zurich (CH)
(72) Inventor: SPENCER, Nicholas, 8702 Zollikon (CH); SIMIC, Rok, 8048 Zürich (CH)

(57) **Abstract**

A method of controlling at least one tribological property of a gel (3) comprising one or more crosslinked polymers, the method comprising the steps of providing at least one source of molecular oxygen (1) that is interfaced with a reaction mixture (2), and producing the gel (3) from the reaction mixture (2) while molecular oxygen from the source of molecular oxygen (1) is present at least at an interface (34) between the reaction mixture (2) and the source of molecular oxygen (1) during at least one period in time.

## Description

### TECHNICAL FIELD

The present invention relates to a method of controlling at least one tribological property of a gel according to claim 1, to the use of such a method for the manufacturing of a medical device according to claim 10, to a gel according to claim 11, to a vessel for controlling at least one tribological property of a gel according to claim 13, to an assembly for controlling at least one tribological property of a gel according to claim 14, and to the use of a source of molecular oxygen for controlling at least one tribological property of a gel according to claim 15.

### PRIOR ART

Gels such as hydrogels or organogels constitute a class of soft materials consisting of a three-dimensional, crosslinked polymer network with large amounts of water or organic solvents tightly incorporated within their structure. It is this structure combining water or an organic solvent with polymers that makes these gels unique among synthetic materials. The polymer network provides water or an organic solvent with form-stability - a property traditionally associated with solids. On the other hand, water or the organic solvent provides the polymer, typically a solid, with the possibility of rapid gas and liquid diffusion together with a low modulus that is otherwise atypical for solids. This combination results in a material that is intermediate in its properties between solids and liquids. Typical characteristics are a high diffusion rate for gases and small molecules, transparency, oxygen permeability, flexibility and lubricity.

With this unique set of properties, the material exhibits similarities to human body tissue like no other existing synthetic material. Due to this resemblance, the applications of gels have often been targeted towards biomedical areas - a challenging field, in which the benefits of the polymeric network structure in combination with a high water content can be utilized to full advantage. The application of gels in the medical sector is a constantly growing field, with diverse current and future applications ranging from spray bandages and contact lenses to intelligent drug-delivery systems, as well as injectable tissue replacements and implants in all parts of the human body.

Many applications of gels require a high degree of lubricity, such as the side of a contact lens that is sliding against the eyelid or the sliding surface of a catheter, whereas other applications necessitate a higher friction, such as the side of a contact lens that is placed upon the eye, or an implant that is designed to stay in one place.

By molding hydrogels against different surfaces, it has been shown that the friction of the hydrogel can be modified. For example, when a glass mold is used, the hydrogel surface has a high friction and is crosslinked, but when a polystyrene (PS) mold is used, the surface has low friction and terminates in loose chains, i.e. it is "brushy", see for example Gong, J.P., Kurokawa, T., Narita, T., Kagata, G., Osada, Y., Nishimura, G., and Kinjo, M. (2001), "Synthesis of hydrogels with extremely low surface friction", Journal of the American Chemical Society, 123(23), 5582-5583 and Meier, Y. A., Kaihuan Zhang, N. D. Spencer, Rok Simic (2019), "Linking Friction and Surface Properties of Hydrogels Molded Against Materials of Different Surface Energies", Langmuir, 2019, 35, 15805-15812. The generally accepted explanation for these findings has been that it is the surface energy that influences this behavior, wherein low-surface-energy molding materials lead to brushy surfaces (low friction), while high-surface-energy materials lead to crosslinked surfaces (high friction). This dogma has been perpetuated in many laboratories since it seemed to work well. On the other hand, understanding why it works has been difficult.

Attempting to understand the working principle the inventors of the present application have coated a glass mold with a hydrophobic monolayer with silane chemistry and determined that it still behaved like glass, producing a crosslinked hydrogel surface. The inventors also plasma treated a polystyrene surface, making it hydrophilic, wherein it still behaved like polystyrene. These findings destroyed the surface-energy dogma.

### SUMMARY OF THE INVENTION

The inventors have surprisingly found out that the presence of molecular oxygen during the generation of a gel comprising one or more crosslinked polymers enables the generation of a gel having at least one tribological property that is controllable over a wide range and that may also be selectively spatially distributed over at least one surface of the gel.

Hence, in a first aspect, a method of controlling at least one tribological property of a gel comprising one or more crosslinked polymers is provided. The method comprises the steps of i) providing at least one source of molecular oxygen that is interfaced with a reaction mixture, and ii) producing the gel from the reaction mixture while molecular oxygen from the source of molecular oxygen is present at least at an interface between the reaction mixture and the source of molecular oxygen during at least one period in time.

The source of molecular oxygen being interfaced with the reaction mixture means that the source of molecular oxygen is in communication with the reaction mixture such that molecular oxygen can flow from the source of molecular oxygen to the reaction mixture. The interface between the reaction mixture and the source of molecular oxygen is understood as a region where the molecular oxygen can impinge or impact on the reaction mixture. As will be explained in greater detail further below, the reaction mixture is preferably provided in a vessel or the like that has at least one surface and that is in communication with the source of molecular oxygen. In this case, the interface can be seen as a surface region, i.e. a region near the surface of the vessel, where the molecular oxygen impinges or impacts on the reaction mixture. As such, it can be said that the method according to the invention enables the controlling of at least one tribological property of at least one surface of a gel comprising one or more crosslinked polymers.

The expression "molecular oxygen" refers to diatomic oxygen, also known as dioxygen or free oxygen or O₂.

The molecular oxygen being present in the reaction mixture during the production of the gel is preferably in its gaseous phase.

The reaction mixture that is used to produce the gel preferably comprises components that are well-known in the art. The gel comprising one or more crosslinked polymers particularly preferably corresponds to a hydrogel or to an organogel. As is well-known in the art, hydrogels are preferably synthesized in a water-based solution and then washed and swollen in pure water. Organogels are preferably made in a solvent-based solution and then washed and swollen in the solvent. Besides, it is noted that the solvent could in principle be swapped out after synthesis. It is also noted that at least some gels can be immersed in several solvents. For instance, hydroxyethyl methacrylate (HEMA) gels can be synthesized in water and then immersed in either water or in ethanol. In summary it can therefore be said that the gel according to the invention is preferably produced from the reaction mixture in a first step, and thereafter is immersed or swollen in water and/or in at least one solvent so as to produce a hydrogel or an organogel in a second step. In particular, and as will be explained further below, the gel that is produced according to the invention is preferably suitable for medical applications and/or for applications on and/or in the human body. The gel is particularly preferably suitable for opthalmological applications and/or for catheter applications and/or for vaginal applications and/or for anal applications. A reaction mixture comprising the corresponding components is thus preferred. In other words, the reaction mixture preferably comprises one or more polymers or copolymers thereof and/or polymerizable agents such as monomers as they are known in the field of the art.

It is furthermore preferred that the gel is produced by a radical polymerization process as it is known in the art. As such, it is preferred that the reaction mixture comprises one or more components that enable the radical polymerization process such as one or more initiators that can initiate the radical polymerization. Exemplary components can be found in US 8 870 372, which discloses conceivable polymers or copolymers thereof, polymerizable agents, and initiators for the production of hydrogel contact lenses.

The at least one tribological property preferably is a frictional property such as a coefficient of friction and/or a wear property such as a coefficient of wear and/or an adhesion of the gel, in particular of the at least one surface of the gel. In the context of the present invention the adhesion refers to the force or energy per unit area needed for a separation of the gel from a reference material. Hence, the method according to the invention preferably enables the controlling of a coefficient of friction and/or of a coefficient of wear and/or of an adhesion of the gel.

The molecular oxygen being present at the interface can be kept constant during the at least one period in time. Alternatively, it is likewise conceivable that the molecular oxygen being present at the interface is varied during the at least one period in time.

The presence and amount of the molecular oxygen, i.e. the concentration of molecular oxygen at the interface, determines the tribological properties of the gel. Hence, varying the concentration of the molecular oxygen allows one to vary the tribological properties of the gel. Moreover, a variation of the molecular oxygen at the interface during the production of the gel enables the generation of a gel whose tribological properties exhibit a gradient and/or pattern, see also further below.

The molecular oxygen can be present at the interface during an entire period of production of the gel or during a part of the entire period of production of the gel. The entire period of production is understood as the time needed for crosslinking the compounds of the reaction mixture so as to generate the gel comprising the crosslinked polymers. In the former case, molecular oxygen is present during the entire time. In the latter case the molecular oxygen is present only temporarily. In the former case as well as in the latter case it is furthermore conceivable that the concentration of the molecular oxygen at the interface is kept constant or, alternatively, is varied. That is, it is conceivable to supply molecular oxygen during the entire period of production, while the concentration of the molecular oxygen is kept constant or is varied. Likewise, it is conceivable to supply molecular oxygen during a part of the entire period of production, while the concentration of the molecular oxygen is kept constant or is varied.

It is furthermore conceivable that molecular oxygen is present during at least one further period in time, and wherein said at least one further period in time follows immediately after the at least one period in time or, alternatively, wherein said at least one further period in time is offset to the at least one period in time, i.e. there can be a time interval between the at least one period in time and the at least one further period in time. A duration of the at least one period in time and the at least one further period in time can be the same or different. Moreover, a concentration of the molecular oxygen at the interface during the at least one period in time and the at least one further period in time can be kept the same or can be set to be different. It should be noted that the above can be applied during one further period in time or during two or more further periods in time. Hence, any statement made with regard to the at least one period in time likewise applies to the case wherein molecular oxygen is present during at least one further period in time or during two or more further periods in time and vice versa.

A concentration of the molecular oxygen at the interface and/or in the source of molecular oxygen is preferably used to control the tribological property of the gel. Additionally or alternatively, a release rate associated with the release of molecular oxygen from the source of molecular oxygen into the reaction mixture is preferably used to control the tribological property of the gel.

As mentioned above, by varying the molecular oxygen being present at the interface the tribological properties can be varied. Hence, the method preferably further comprises the step of varying a concentration of the molecular oxygen at the interface and/or a concentration of the molecular oxygen in the source of molecular oxygen and/or a release rate associated with the release of molecular oxygen into the reaction mixture during the at least one period in time.

The source of molecular oxygen is preferably provided by one or more fluids comprising molecular oxygen and/or by one or more chemical reactions producing molecular oxygen and/or by one or more electrolytic systems producing molecular oxygen and/or by one or more compounds comprising molecular oxygen and being configured to release the molecular oxygen.

The fluid preferably comprises or consists of one or more liquids and/or of one or more gases. A preferred gas comprises or consists of molecular oxygen. Another preferred gas comprises or consists of air. Another preferred gas comprises molecular oxygen and molecular nitrogen. Further gases or mixtures of gases that comprise molecular oxygen are of course likewise conceivable and are well-known to the skilled person. It is however likewise conceivable to provide a source of molecular oxygen that comprises or consists of one or more liquids. Such a source of molecular oxygen could be provided by water, for example. However, it should be noted that molecular oxygen is soluble in many fluids, including water but also other solvents such as fluorinated solvents, e.g. perfluorodecalin. That is, it is furthermore preferred to provide molecular oxygen from a source of molecular oxygen that corresponds to a mixture of one or more liquids and one or more gases. Moreover, a controlling device being configured to control a concentration of molecular oxygen in one or more liquids such as a bubbler system could be used. In other words, the controlling device enables the production of one or more liquids that comprise molecular oxygen at a defined level. The controlling device is preferably configured to dissolve a particular amount of molecular oxygen in one or more liquids. For instance, the controlling device could comprise a liquid such as water and air or nitrogen, wherein the air is preferably used to increase the concentration of molecular oxygen in the source of molecular oxygen and nitrogen is used to decrease the concentration of molecular oxygen in the source of molecular oxygen. That is, by supplying a fluid that does not comprise molecular oxygen to the source of molecular oxygen the concentration of the molecular oxygen in the source of molecular oxygen can be reduced.

The concentration of the molecular oxygen in the fluid can be used to control the tribological property of the gel. Namely, the concentration of the molecular oxygen is determined by a release rate at which the molecular oxygen is released from the fluid into the reaction mixture, whereby the tribological property of the gel is controlled.

However, as mentioned, other sources of molecular oxygen are likewise conceivable. For example, it is conceivable to produce molecular oxygen during one or more chemical reactions. In other words, the molecular oxygen can be provided by a chemical reaction that produces molecular oxygen, e.g. by the provision of oxygen-generating compounds such as peroxides, for example hydrogen peroxide, superoxides, chlorates, perchlorates, ozonides, etc. Further compounds such as catalysts and/or enzymes promoting the decomposition and the release of molecular oxygen could be additionally provided. Such oxygen-producing chemical reactions are well-known in the art. For instance, they find application in chemical oxygen generators, see e.g. US 4 548 730.

Examples of electrolytic systems that produce molecular oxygen are also well-known in the art. For example, electrolytic polymers such as could be used. The provision of solid oxides as they are used in fuel cells that release molecular oxygen in the presence of a potential are likewise conceivable. As an example, EP 2 148 941 A1 discloses the electrolytical generation of molecular oxygen from water.

Compounds comprising molecular oxygen and being configured to release the molecular oxygen are likewise conceivable. Such compounds are preferably saturated with molecular oxygen, for example by exposing them to air or another oxygen-rich fluid. To this end, a variety of compounds are conceivable. For instance, the compound could comprise or consist of one or more polymers or copolymers thereof such as polyethylene and/or polystyrene and/or polytetrafluoroethylene and/or polypropylene and/or polyvinyl chloride, etc., and wherein said polymers or copolymers thereof are saturated with molecular oxygen.

The source of molecular oxygen can be arranged separately from the reaction mixture. For example, it is conceivable to provide the source of molecular oxygen in a confined space such as a reservoir, container, tube or the like, and wherein said confined space is in fluid communication with the reaction mixture. As mentioned initially, the reaction mixture is preferably provided in a vessel or the like. Hence, a conceivable fluid communication is provided between the confined space comprising the fluid and the vessel comprising the reaction mixture. To this end the confined space and the vessel can be in direct contact with one another. However, an indirect arrangement is likewise conceivable, for example by providing at least one fluidic element as they are known in the art, e.g. a tube, pipe or the like that connects the confined space with the vessel. The fluid can be flowing, for example along the reservoir, container, tube or the like. However, it is likewise conceivable that the fluid is steady, i.e. not flowing along a flowing direction but merely kept within the confined space. If the fluid is flowing a mass flow rate of the fluid and/or a flow velocity of the fluid can be used to maintain a concentration of the molecular oxygen in the fluid constant or to allow changes. The same applies with regard to the other conceivable sources of molecular oxygen. That is, it is conceivable to provide compounds capable of undergoing chemical reactions producing molecular oxygen and/or electrolytic systems producing molecular oxygen and/or compounds being configured to release the molecular oxygen in a confined space as just described.

Alternatively, the source of molecular oxygen can be arranged at least partially in contact with the reaction mixture. For example, fluid comprising or consisting of molecular oxygen could be passed over a vessel or the like comprising the reaction mixture. Furthermore, compounds capable of undergoing chemical reactions producing molecular oxygen and/or electrolytic systems producing molecular oxygen and/or compounds being configured to release molecular oxygen could be arranged at least partially in a vessel or the like, for example by arranging them on a surface of the vessel. Compounds being configured to release molecular oxygen and being arranged at least partially in contact with the reaction mixture can correspond to so-called insert elements, see further below.

The method preferably further comprises the step of providing at least one separation element that separates the source of molecular oxygen from the reaction mixture. In particular, when the source of molecular oxygen is arranged separately from the reaction mixture, it is preferred that at least one separation element provides this separation. For example, at least one separation element could be arranged between the confined space such as the reservoir, container, tube or the like comprising the fluid and the vessel comprising the reaction mixture. In this case the at least one separation element is particularly preferably arranged at least partially on a surface of the vessel and/or the at least one separation element particularly preferably at least partially provides the surface of the vessel.

The separation element preferably is at least partially oxygen-permeable. It is furthermore preferred that the separation element has an oxygen permeability of at least 10 cm³·25µm/(m²·24h·atm), preferably of at least 100 cm³·25µm/(m²·24h·atm), more preferably of at least 1000 cm³·25µm/(m²·24h·atm). Additionally or alternatively it is preferred that an oxygen permeability of the separation element is in the range of 10 cm³·25µm/(m²·24h·atm) to 10'000 cm³·25µm/(m²·24h·atm). Moreover, the separation element preferably comprises or consists of one or more polymers or copolymers thereof such as polyethylene and/or polystyrene and/or polytetrafluoroethylene and/or polypropylene and/or polyvinyl chloride. The separation element preferably corresponds to a membrane or layer or the like. A thickness of the separation element can be constant over a length of the separation element. Alternatively, a thickness of the separation element can vary along the length of the separation element. A thickness of the separation element preferably is at least 1 nanometer or more, more preferably at least 100 nanometer or more. Additionally or alternatively a thickness of the separation element preferably is in the range of 1 nanometer to 1000 micrometer.

The properties of the separation element, for example its thickness or oxygen-permeability, can be used to determine a concentration of molecular oxygen being present in the reaction mixture as they can determine how fast molecular oxygen can be supplied from the source of molecular oxygen into the reaction mixture. In other words, the separation element allows controlling how fast molecular oxygen is released into the reaction mixture.

The method preferably further comprises the step of providing at least one further separation element, wherein the further separation element is arranged at a location being different from a location of the separation element, and/or wherein the further separation element has one or more properties such as a thickness and/or oxygen permeability that differs from one or more properties of the separation element.

For instance, a first separation element could be arranged and/or configured so as to produce a first part of the gel having a first tribological property and a second separation element being different from the first separation element could be arranged and/or configured so as to produce a second part of the gel having a second tribological property being different from the first tribological property. For example, the first separation element could be arranged and/or configured so as to generate a first surface of the gel having a coefficient of wear and/or a coefficient of friction and/or an adhesion being different from the coefficient of wear and/or the coefficient of friction and/or the adhesion of a second surface of the gel being generated by the second separation element. The provision of two or more separation elements therefore enables the generation of a gel with tribological properties that exhibit a gradient and/or a pattern.

As will be explained in greater detail further below, various applications of the gel produced according to the invention are conceivable. For example, the gel could be used for the manufacturing of contact lenses, and wherein the provision of different separation elements could be used to impart different coefficients of friction to different sides of the lens. Hence, the first and second surfaces of the gel mentioned above could correspond to the opposing surfaces of a contact lens, wherein one surface of the lens has a high degree of lubricity such that it can easily slide against the eyelid and the other surface of the lens has a high coefficient of friction such that it stays in place upon the eye.

Various arrangements of the one or more separation elements are conceivable. For example, two or more separation elements could be arranged immediately adjacent to one another or spaced apart from one another. Additionally or alternatively, two or more separation elements could be arranged at least partially overlapping one another. Additionally or alternatively two or more separation elements could be arranged opposite to each another, for example on opposing surfaces of a vessel comprising the reaction mixture. Additionally or alternatively two or more separation elements could be arranged offset with respect to one another. As mentioned initially, these two or more separation elements can be the same or different from one another with regard to their properties such as a thickness and/or oxygen permeability and/or composition, etc.

As mentioned earlier, the source of molecular oxygen can be provided by at least one insert element that is at least partially in contact with the reaction mixture. The insert element preferably comprises or consists of one or more compounds comprising molecular oxygen and being configured to release molecular oxygen. That is, the insert element can serve the purpose of providing the source of molecular oxygen. The insert element is preferably oxygen-saturated, for example by exposing it to air or another oxygen-rich fluid. As already mentioned, a variety of compounds constituting the insert element are conceivable. For instance, insert element could comprise or consist of one or more polymers or copolymers thereof such as polyethylene and/or polystyrene and/or polytetrafluoroethylene and/or polypropylene and/or polyvinyl chloride, etc.

The insert element can correspond to a layer or membrane. A thickness of the insert element can be constant over a length of the insert element. Alternatively, a thickness of the insert element can vary along the length of the insert element. A thickness of the insert element preferably is at least 1 nanometer or more, more preferably at least 100 nanometer or more. Additionally or alternatively a thickness of the insert element preferably is in the range of 1 nanometer to 1000 micrometer.

The properties of the insert element, for example its thickness or oxygen-permeability or its oxygen-saturation, can be used to determine a concentration of molecular oxygen being present in the reaction mixture as they can determine how much molecular oxygen is released into the reaction mixture. For example, an insert element having a larger thickness enables a larger amount of molecular oxygen to diffuse into the reaction mixture so it thus affects the concentration of molecular oxygen in the reaction mixture.

The method preferably further comprises the step of providing at least one further insert element, wherein the further insert element is arranged at a location being different from a location of the insert element, and/or wherein the insert element has one or more properties such as a thickness and/or oxygen permeability and/or an oxygen-saturation that differs from one or more properties of the insert element.

As in the case of the first and second separation elements, at least a first insert element could be arranged and/or configured so as to produce a first part of the gel having a first tribological property and at least a second insert element being different from the first insert element could be arranged and/or configured so as to produce a second part of the gel having a second tribological property being different from the first tribological property. For example, the first insert element could be arranged and/or configured so as to generate a first surface of the gel having a coefficient of wear and/or a coefficient of friction and/or an adhesion being different from the coefficient of wear and/or the coefficient of friction and/or the adhesion of a second surface of the gel being generated by the second insert element. The provision of two or more insert elements therefore enables the generation of a gel whose tribological properties exhibit a gradient and/or pattern, see also further below.

Thus, depending on the desired application of the gel produced according to the method, the provision of different insert elements could be used to manufacture contact lenses having different coefficients of friction on the opposing sides of the lens, etc.

Various arrangements of the one or more insert elements are conceivable. For example, two or more insert elements could be arranged immediately adjacent to one another or spaced apart from one another. Additionally or alternatively, two or more insert elements could be arranged at least partially overlapping one another. Additionally or alternatively, two or more insert elements could be arranged opposite to each another, for example on opposing surfaces of a vessel comprising the reaction mixture. Additionally or alternatively, two or more insert elements could be arranged offset with respect to one another. As mentioned initially, these two or more insert elements can be the same or different from one another with regard to their properties such as thickness and/or oxygen-saturation and/or oxygen-permeability and/or composition, etc.

The method preferably further comprises the step of at least partially blocking a supply of molecular oxygen into the reaction mixture, preferably by providing at least one blocking element. The at least one blocking element is particularly preferably arranged at the interface between the reaction mixture and the source of molecular oxygen.

That is, the supply of molecular oxygen into the reaction mixture can be at least partially blocked. Said blocking is particularly preferably carried out spatially, for example by arranging two or more blocking elements at one or more locations in a vessel comprising the reaction mixture and/or at one or more locations on the at least one separation element and/or at one or more locations on the at least one insert element, etc. In this way a variation, in particular a local or spatial variation, of the presence and/or concentration of molecular oxygen at the interface and thus of the at least one tribological property is achieved. Conceivable blocking elements are at least partially, preferably entirely oxygen-impermeable. A preferred blocking element comprises or consists of glass and/or of one or more oxygen-impermeable polymers or copolymers thereof and/or of one or more metal compounds. An oxygen-permeable polymer is polyvinylidene chloride. One or more metal compounds could be applied by sputtering, for example over at least a region of an oxygen-permeable element such as the insert element and/or the separation element.

The method preferably further comprises the steps of providing the reaction mixture in a mold and producing the gel by molding the reaction mixture while molecular oxygen is present at least at an interface between the mold and the reaction mixture.

That is, the gel is preferably produced by molding. The vessel comprising the reaction mixture as mentioned above thus preferably corresponds to a mold.

The interface between the mold and the reaction mixture is understood as a region near a surface of the mold, where the molecular oxygen impinges or impacts on the reaction mixture.

The mold preferably comprises one or more of the components as described above, e.g. the mold preferably comprises one or more separation elements and/or one or more insert elements and/or one or more blocking elements. One or more of these components can be arranged in or on a part of the mold, e.g. in or on a surface of the mold. It is however likewise conceivable that one or more of these components constitute a part of the mold. For example, a bottom of the mold could be formed by one or more separation elements.

Consequently, the gel is preferably produced by molding the reaction mixture in the mold, and wherein a presence of the oxygen and/or a concentration of the oxygen being released from the mold, in particular from at least a part of at least one surface of the mold, is used to control the tribological property of the gel, in particular the tribological property of at least one surface of the gel being in contact with the at least one surface of the mold. Likewise, the gel is preferably produced by molding the reaction mixture in the mold, and wherein a presence of the oxygen and/or a concentration of the oxygen being diffused through the mold, in particular through at least a part of at least one surface of the mold, is used to control the tribological property of the gel, in particular the tribological property of at least one surface of the gel being in contact with the at least one surface of the mold.

In another aspect the method as described above is used for the manufacturing of a medical device and/or of a device being suitable for applications on and/or in the human or animal body. The medical device preferably is an ophthalmological device such as a contact lens and/or a catheter. The device being suitable for applications on and/or in the human or animal body preferably is a condom.

The gel according to the invention is thus preferably suitable for medical applications and/or for applications on and/or in the human body. The gel is particularly preferably suitable for opthalmological applications and/or for catheter applications and/or for vaginal applications and/or for anal applications. Various applications of a catheter are conceivable, for example catheters used in urology or for angioplasty in the heart or brain. It is furthermore preferred that the gel according to the invention is sterile.

In a further aspect a medical device, in particular an ophthalmological device and/or a catheter produced according to the method as described above is provided. The ophthalmological device preferably is a contact lens.

In a further aspect a device for applications on and/or in the human or animal body being produced according to the method as described above is provided, the device preferably is a condom.

In a further aspect a gel comprising one or more crosslinked polymers is provided, wherein
the gel comprises at least a first surface region and a second surface region, wherein the first surface region is associated with at least one first tribological property and the second surface region is associated with at least one second tribological property, and wherein the first tribological property and the second tribological property are different from one another.

The first surface region and the second surface region can be provided on a same surface of the gel or on different surfaces of the gel.

The difference between the first tribological property and the second tribological property can be a difference in the magnitude of a same tribological property. For example, the first and second tribological properties could both correspond to a coefficient of friction, and wherein the difference lies in one of the coefficients of friction being larger than the other. Additionally or alternatively, it is conceivable that the difference between the first tribological property and the second tribological property lies in the type of the tribological property. For example, the first tribological property could correspond to a particular coefficient of friction and the second tribological property could correspond to a particular coefficient of wear.

A difference between the first tribological property and the second tribological property preferably is at least one order of magnitude.

For example, a conceivable range of the coefficients of friction is between 0.001 to 1, more preferably between 0.01 to 0.1. Hence, a first coefficient of friction constituting the first tribological property could be 0.01 and a second coefficient of friction constituting the second tribological property could be 0.1. It should be noted that these values are merely given as one conceivable numerical example. Other values are of course likewise conceivable.

The first surface region and the second surface region can be arranged adjacent to one another or at a distance from one another. For example, the first surface region could immediately follow the second surface region. Alternatively, it is conceivable that the first surface region is spatially spaced from the second surface region.

The gel preferably further comprises a pattern that is constituted by at least one or more first surface regions and one or more second surface regions. That is, the surface regions are preferably arranged in a pattern.

At least one or more first surface regions and one or more second surface regions are preferably arranged such that their associated first and second tribological properties exhibit a gradient.

Within the context of the present invention, a pattern being constituted by at least one or more first surface regions and one or more second surface regions comprises spatially discrete areas of different tribological properties, e.g. of different coefficients of friction. Within these areas, the particular tribological property is constant. Within the context of the present invention, a gradient in the tribological properties is understood as tribological properties that change preferably continuously with distance, in 1 or 2 dimensions. It should be noted that patterns and/or gradients can be over any length scale.

In a further aspect a vessel for controlling at least one tribological property of a gel comprising one or more crosslinked polymers is provided. The vessel is configured to receive a reaction mixture. The vessel is further configured to be interfaced with at least one source of molecular oxygen. The vessel is further configured such that the gel is produced from the reaction mixture while molecular oxygen from the source of molecular oxygen is present at least at an interface between the reaction mixture and the source of molecular oxygen during at least one period in time.

The vessel is preferably configured to be used in the method as described above. It is furthermore preferred that the vessel comprises one or more of the components as described above. As such, any statements and explanations made with respect to the method likewise apply to the vessel and vice versa. For example, the vessel preferably comprises one or more separation elements and/or one or more insert elements and/or one or more blocking elements. One or more of these components can be arranged in or on a part of the vessel, e.g. in or on a surface of the vessel. It is however likewise conceivable that one or more of these components constitute a part of the vessel. For example, a bottom of the vessel could be formed by a separation element.

The vessel particularly preferably corresponds to a mold that is configured for being used in a molding process as it is known in the art.

In a further aspect, an assembly for controlling at least one tribological property of a gel comprising one or more crosslinked polymers is provided. The assembly comprises at least one vessel, in particular a mold, that is configured to receive a reaction mixture. The assembly further comprises at least one source of molecular oxygen that is interfaced with the at least one vessel. The assembly is configured such, that the gel is produced from the reaction mixture while molecular oxygen from the source of molecular oxygen is present at least at an interface between the reaction mixture and the source of molecular oxygen during at least one period in time.

The assembly is preferably configured to perform the method as described above. It is furthermore preferred that the assembly comprises one or more of the components as described above. As such, any statements and explanations made with respect to the method and/or the vessel likewise apply to the assembly and vice versa. For example, it is preferred that the vessel corresponds to a vessel as described above and/or that the source of molecular oxygen corresponds to a source of molecular oxygen as described above. Furthermore, the vessel and the source of molecular oxygen are preferably in communication with one another as described above. That is, they can be arranged separately from one another and being in communication with one another, for example via at least one separation element. Alternatively, the source of molecular oxygen can be arranged at least partially in the vessel.

In a further aspect, a source of molecular oxygen is used for controlling at least one tribological property of a gel comprising one or more crosslinked polymers.

The source of molecular oxygen is preferably configured to be interfaced with a reaction mixture, particularly preferably with a vessel comprising a reaction mixture. The source of molecular oxygen is preferably configured such, that the gel is produced from the reaction mixture while the molecular oxygen is present at least at an interface between the reaction mixture and the source of molecular oxygen during at least one period in time.

The source of molecular oxygen particularly preferably corresponds to a source of molecular oxygen as described above. Consequently, any statements provided previously likewise apply here as well.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1a: shows a perspective view of an assembly comprising a vessel and a source of molecular oxygen according to the invention;
- Fig. 1b: shows an exploded view of the assembly according to figure 1a;
- Fig. 2: shows a diagram depicting force-indentation curves of gels according to the invention that have been produced while different amounts of molecular oxygen were present;
- Fig. 3: shows a diagram depicting coefficients of friction for different sliding speeds of the gels according to figure 2;
- Fig. 4: depicts a schematic view of a vessel comprising a reaction mixture and different sources of molecular oxygen;
- Fig. 5: shows a diagram depicting force-indentation curves of gels that were produced in the vessel according to figure 4;
- Fig. 6: shows a diagram depicting coefficients of friction for different sliding speeds of the gels that were produced in the vessel according to figure 4;
- Fig. 7a: shows a diagram depicting coefficients of friction at two different sliding speeds over several rotations of a gel that was produced according to figure 4;
- Fig. 7b: shows a detailed view of the diagram according to figure 7a;
- Fig. 8a: shows a partial sectional view of a vessel having an oxygen-permeable separation element and comprising a reaction mixture;
- Fig. 8b-8e: show partial sectional views of the vessel according to figure 8a comprising gels that were produced from the reaction mixture, wherein no additional component has been added to the vessel (b), wherein an insert element being oxygen-permeable has been added to the vessel (c), wherein a blocking element being oxygen-impermeable has been added to the vessel (d), and wherein the oxygen-permeable separation element of figure 8a has been replaced by an oxygen-impermeable blocking element (e);
- Fig. 9: shows a diagram depicting force-indentation curves of the gels that were produced in the vessels according to figures 8b to 8e;
- Fig. 10: shows a diagram depicting the coefficient of friction for different sliding speeds of the gels that were produced in the vessels according to figures 8b to 8e;
- Fig. 11: shows a diagram depicting force-indentation curves of further gels according to the invention;
- Fig. 12: shows a diagram depicting the coefficient of friction for different sliding speeds of the gels according to figure 11;
- Fig. 13: shows a diagram depicting force-indentation curves of further gels according to the invention;
- Fig. 14: shows a diagram depicting the coefficient of friction for different sliding speeds of the gels according to figure 13;
- Fig. 15: shows a sectional view of another assembly comprising a vessel and a source of molecular oxygen according to the invention;
- Fig. 16: shows a sectional view of another assembly comprising a vessel and a source of molecular oxygen according to the invention;
- Fig. 17: shows a sectional view of another assembly comprising a vessel and a source of molecular oxygen according to the invention.

### DESCRIPTION OF PREFERRED EMBODIMENTS

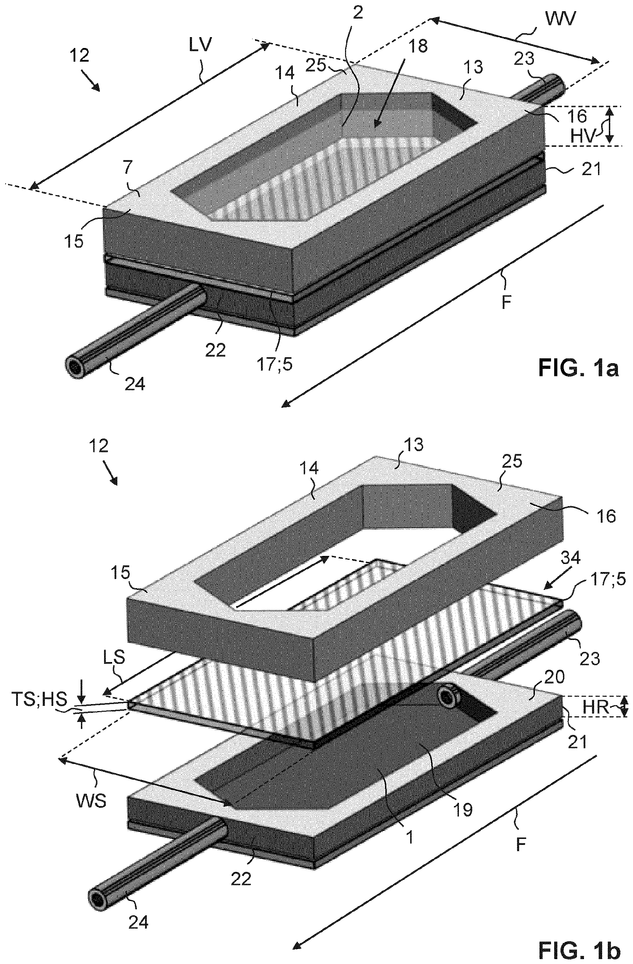

Aspects of the present invention are now illustrated by means of figures 1a to 17. In particular, and as already mentioned, the present invention is based on the insight that molecular oxygen allows controlling of the tribological properties of gels 3 comprising one or more crosslinked polymers. In the following, it will be demonstrated that the release of molecular oxygen into a reaction mixture 2 being comprised in a vessel 7 such as a mold during polymerization of the reaction mixture 2 leads to reduced crosslinking and lower friction at the gel surface. This presents the opportunity for controlling the tribological properties precisely on a gel surface.

Figures 1a and 1b depict a conceivable assembly 12 for the production of such gels 3. As readily follows from said figures, the assembly 12 comprises a vessel 7 and a source of molecular oxygen 1. In the present example the vessel 7 corresponds to a mold that can be subjected to a molding process. The vessel 7 comprises four side walls 13-16 being formed in one piece and a bottom wall 17 that is attached to said four side walls 13-16. Here, the bottom wall 17 corresponds to an oxygen-permeable element that allows a diffusion of oxygen into a reaction chamber 18 being delimited by the four side walls 13-16 and the bottom wall 17. A reaction mixture 2 is received in the reaction chamber 18. The source of molecular oxygen 1 is provided by a fluid that is comprised in a confined space, which confined space comprises here a reservoir 19. The reservoir 19 has an open upper side 20 and, with said open upper side 20, the reservoir 19 is in fluid communication with the vessel 7. In particular, the reservoir 19 is in fluid communication with the vessel 7 via the oxygen-permeable bottom wall 17 of the vessel 7. Hence, the bottom wall 17 constitutes a separation element 5 that separates the vessel 7, in particular the reaction chamber 18 of the vessel 7, from the source of molecular oxygen 1. Two opposing lateral side walls 21, 22 of the reservoir 19 are pierced by a tube 23, 24. Said tubes 23, 24 serve the purpose of allowing the fluid to flow. In fact, the fluid flows here from a first tube 23 into the reservoir 19, through said reservoir 19 and out of the reservoir 19 via a second tube 24 along a flowing direction F. The assembly 12 is configured such, that the gel 3 is produced from the reaction mixture 2 while molecular oxygen from the source 1 is present at least at an interface 34 between the reaction mixture 2 and the source of molecular oxygen 1. The interface 34 between the reaction mixture 2 and the source of molecular oxygen 1 corresponds to the region where the molecular oxygen can impinge or impact on the reaction mixture 2. In the present example, the interface 34 corresponds to a surface region, here to a region near the bottom wall 17 or the separation element 5 of the vessel 7, where the molecular oxygen impinges or impacts on the reaction mixture 2. In the depicted example the vessel 7 has a width WV of 20 millimeter and a length LV of 50 millimeter, The four sidewalls 13-16 of the vessel 7 comprise a thermoplastic, in particular a transparent thermoplastic such as poly(methyl methacrylate). A height HV of the vessel 7 is 5 millimeter. The separation element 5 is of a same width WS and length LS as the vessel 7. A height HS of the separation element is 10 micrometer. Said height HS can also be referred to as a thickness TS of the separation element 5. The height HS or thickness TS of the separation element 5 is here constant over the entire length LS and width WS of the separation element 5. Thus, the separation element 5 has the shape of a constant membrane or layer. In the present example the separation element 5 consists of polyethylene (PE). The reservoir 19 comprising the fluid 1 has the same lateral dimensions as the vessel 7, wherein a height HR of the reservoir is 2 millimeter.

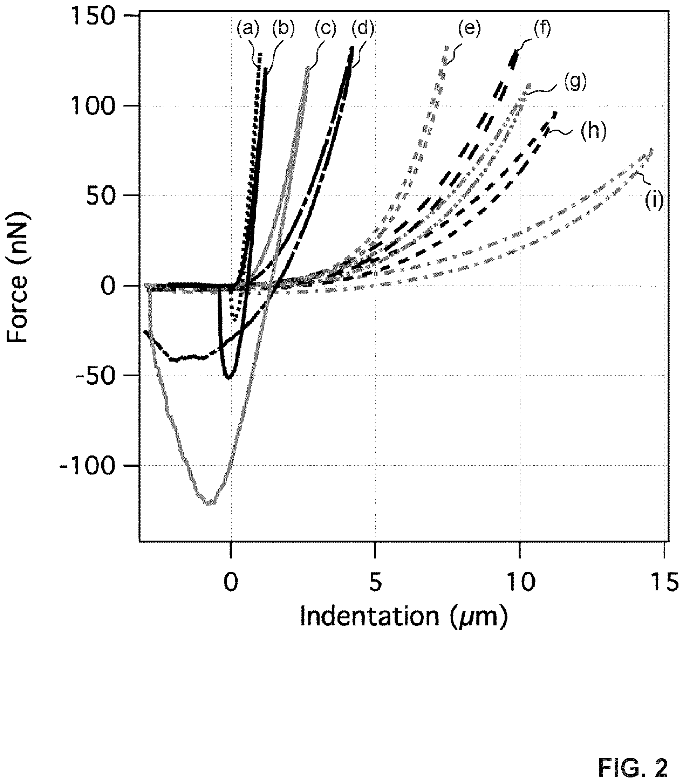

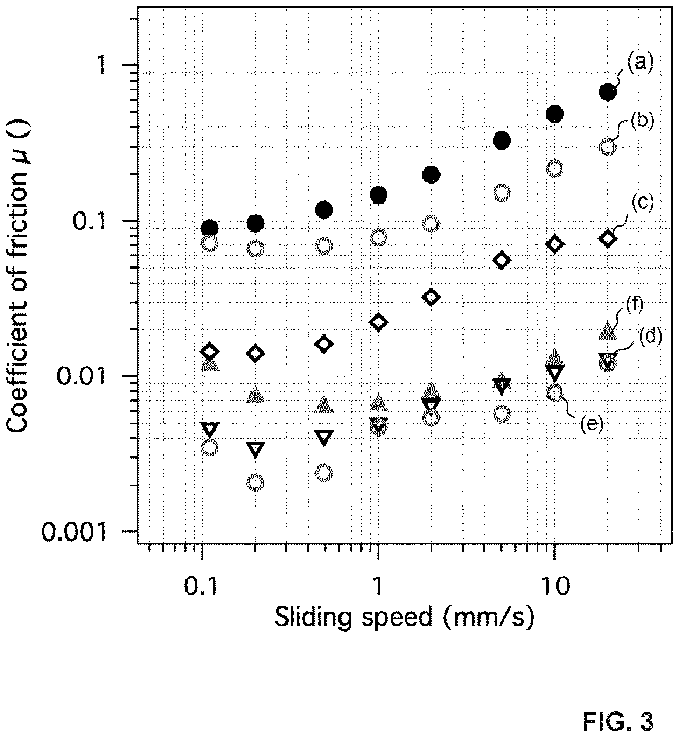

With reference to figures 2 and 3 different aspects of gels 3 prepared from a first reaction mixture 2 and in the assembly 12 of figures 1a and 1b are discussed. Said first reaction mixture 2 consisted of 4 milliliter of an aqueous polymerizing solution consisting of 10 wt% acrylamide, 0.4 wt% bis-acrylamide and 0.01 wt% lithium phenyl-2,4,6-trimethylbenzoylphosphinate (LAP) as an ultraviolet-light-triggered photoinitiator. During the preparation of these gels 3 the source of molecular oxygen 1 was provided by a mixture of fluids, namely a mixture of air and nitrogen, and wherein a concentration of the molecular oxygen was varied between 0.0% (pure nitrogen) and 22% (pure air). The fluids 1 were allowed to flow along the flowing direction F with a flow rate of 5 ml.min⁻¹. After the establishment of the desired molecular oxygen concentration in the reservoir 19, the entire vessel 7 was exposed from an upper side 25 of the vessel 7 to a UV source of 365 nm, 1.4 mW.cm⁻² for a period of 20 minutes, after which the gel 3 (approximately 4 mm in thickness) was demolded and rinsed in deionized water. The molding process was repeated with different oxygen concentrations in the flowing fluid 1, to produce a set of gels 3. A control sample was prepared, in which the separation element 5, i.e. the polyethylene membrane, was replaced by a blocking element 6 that is oxygen-impermeable, here with a glass sheet having a thickness of 0.15 millimeter.

Figure 2 shows a diagram depicting force-indentation curves of these gels 3, wherein the gels 3 were investigated by means of nanoindentation, using an atomic force microscope (MFP-3D, Asylum Research, Santa Barbara, USA). The investigated surface of the gel 3 was the one that had been in contact with the bottom wall 17 of the vessel 7, i.e. with the polyethylene membrane constituting the separation element 5 or the glass sheet during the polymerization process. The nanoindentation was carried out in deionized water (MilliQ) at 25° C using a gold-coated tipless cantilever, onto which an 11-µm silica microsphere had been mounted. The spring constant of the microsphere-cantilever assembly was calibrated using the Sader method (Sader J E, Chon J W M, Mulvaney P. Calibration of rectangular atomic force microscope cantilevers. Rev Sci Instrum 70(10): 3967-3969 (1999)) and was corrected for the colloid position to yield a k value of 1.65 N.m⁻¹. The optical-lever sensitivity of the measurement system was determined by pressing the assembly against a silicon wafer in water. Indentation was carried out at 1 µm.s⁻¹ at 40 different locations on each sample. In particular, figure 2 shows representative force-indentation curves for the gel surfaces synthesized against a glass surface (a) and against the PE membrane exposed to flowing pure nitrogen (b), nitrogen containing with 0.2 % O₂ (c), nitrogen with 0.4 % O₂ (d), nitrogen with 1 % O₂ (e), nitrogen with 2 % O₂ (f), nitrogen with 7 % O₂ (g), nitrogen with 14% O₂ (h) and nitrogen with 22 % O₂ (i). The elastic modulus within the first 1 micrometer of depth for the reference glass-molded sample was 30 ± 1 kPa. The elastic moduli of the sample synthesized against the PE membrane with pure nitrogen, nitrogen with 0.2 and 0.4 O₂ were 21 ± 1 kPa, 7.1 ± 0.6 kPa and 3 ± 0.1 kPa, respectively. Increasing the O₂ concentration in the reaction mixture above 1 % resulted in elastic moduli at the surface of the gel below 1 kPa. The results indicate that the indentation behavior of the differently prepared gels varies widely, and the modulus decreases monotonically with increasing concentration of molecular oxygen in the flowing fluid.

Figure 3 shows a diagram depicting coefficients of friction for different sliding speeds of these gels 3. In particular, the frictional properties of the gels 3 were investigated in deionized (MilliQ) water at 25° C by means of reciprocating pin-on-disk experiments (Tribometer: CSM, Needham, MA, USA). The investigated surface of the gel was the one that had been in contact with the bottom wall 17 of the vessel 7, i.e. with the polyethylene membrane constituting the separation element 5 during the polymerization process. The pin consisted of a polyacrylamide gel (diameter 10 mm) that had been molded against glass. The applied load was 0.5 N, which corresponds to a contact pressure of 6 kPa, and the stroke length was 20 mm. The sliding speed over the central 20% of the stroke was varied from 0.1 - 20 mm.s⁻¹ and frictional measurements were averaged over the last five cycles, within this central portion of the stroke only. Figure 3 shows the coefficient of friction as a function of the sliding speed for PE-membrane-molded gels 3 with flowing nitrogen containing 0.0 % O₂ (a), 0.2 % O₂ (b), 0.4 % O₂ (c), 1 % O₂ (d), 7 % O₂ (e) and 22 % O₂ (f). The measurements show a clear correlation of the friction coefficient with the concentration of the molecular oxygen in the flowing fluid 1, with at least an order of magnitude difference between the 0.0 % molecular oxygen case and the gels 3 produced above 1% molecular oxygen.

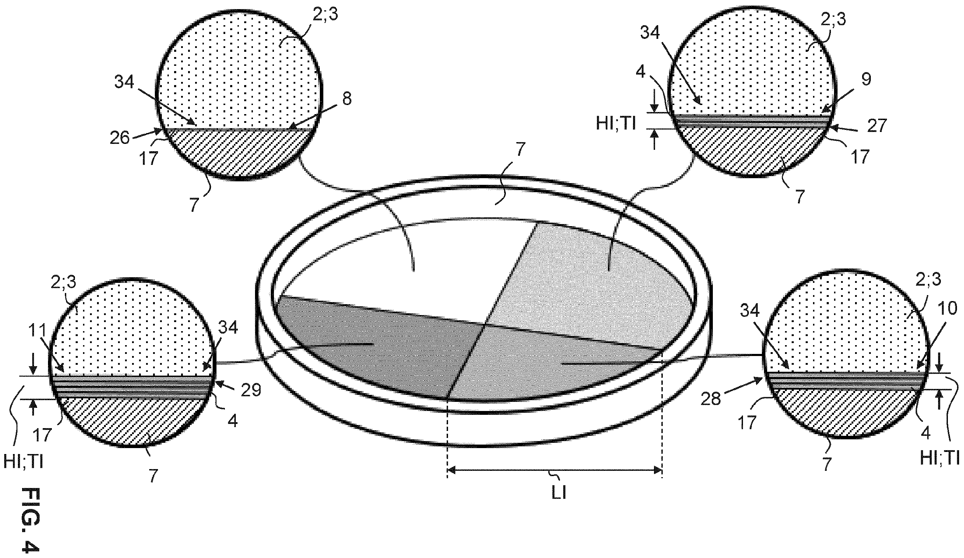

Figure 4 depicts a vessel or mold 7 consisting of a circular glass dish (120 mm in diameter), into which a stack of partially overlapping 10 µm sheets of air-equilibrated, i.e. saturated with molecular oxygen at ambient air conditions, polyethylene PE as a source of molecular oxygen 1 had been placed. These sheets of PE 1 are referred to as insert elements 4 being configured to release molecular oxygen. That is, the insert elements 4 serve as a source of molecular oxygen 1. The stacks of insert elements 4 differ in their heights HI or thicknesses TI. As such, a bottom region of the vessel 7 can be divided into different surface regions, wherein a first surface region 26 comprises the glass dish 7 only, a second surface region 27 comprises a stack of insert elements 4 having a total thickness of 20 µm, a third surface region 28 comprises a stack of insert elements 4 having a total thickness of 30 µm, and a fourth surface region 29 comprises a stack of insert elements 4 having a total thickness of 40 µm. In the depicted example each stack of insert elements 4 has a height HI or thickness TI that is constant over an entire length LI of the insert element 4. Here, 40 milliliter of the polymerization solution, i.e. the first reaction mixture 2 described above, were poured into this vessel 7. The polymerization was carried out as described above with reference to figure 2. The usage of a vessel 7 comprising different surface regions 26-29 results in the formation of different interfaces 34 and ultimately in gels 3 comprising different surface regions 8-11 as well. In fact, said surface regions 8-11 of the gel 3 differ in their tribological properties, see explanations below. The different surface regions 26-29 of the vessel 7 are provided here in the form of a pattern. Consequently, the different surface regions 8-11 of the gel 3 are provided in the form of a corresponding pattern as well. In particular, the pattern of the gel 3 comprises four surface regions 8-11 which are arranged adjacent to one another.

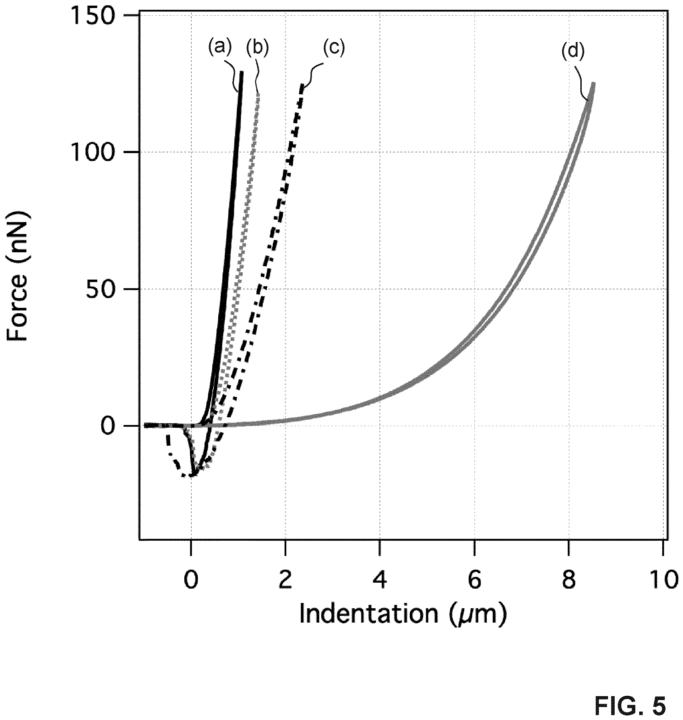

Indentation measurements were carried out as described with reference to figure 2. Figure 5 shows representative indentation curves for the different surface regions 8-11 of the gel 3 that were synthesized against the bare glass surface (a) and against a glass surface covered by a stack of air-equilibrated PE 4 with a total thickness of 20 µm (b), 30 µm (c) and 40 µm (d). The elastic moduli within the first 1 µm of indentation depth for the bare, 20-µm, 30-µm and 40-µm case were 30 ± 1 kPa, 17 ± 1 kPa, 8 ± 5 kPa and <1 kPa, respectively. Indentation results showed that the modulus measured in different surface regions 8-11 of the gel surface that had polymerized against the bottom of the mold 7 depended on the total thickness of PE 4 that had been present in that region, with lower modulus corresponding to higher total thickness of PE 4.

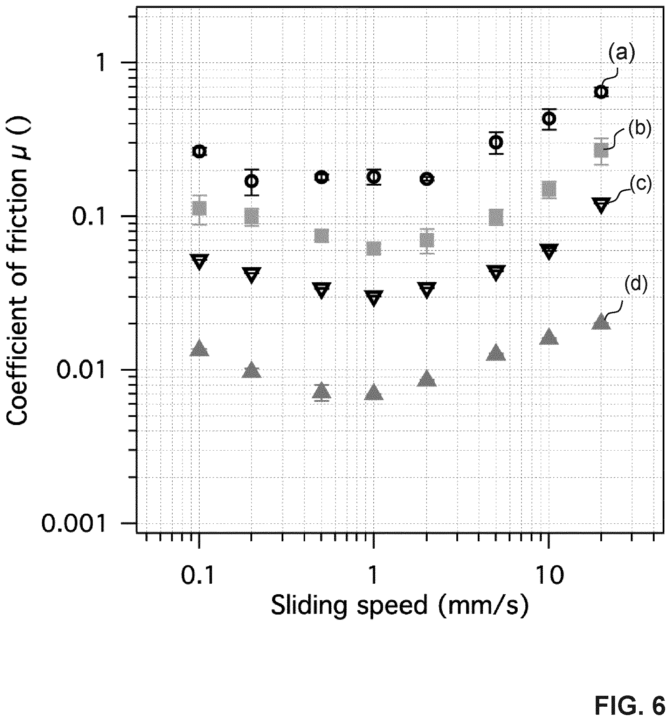

Friction results, carried out as described in the context of figure 3, measuring surface regions 8-11 of the gel 3 that had been molded against 0 µm PE, i.e. against the bare glass surface (a), 20 µm PE (b), 30 µm PE (c) and 40 µm PE (d), are shown in Figure 6. The results showed that friction also depended on the PE 4 thickness TI or height HI, varying over an order of magnitude from 0 µm PE to 40 µm PE.

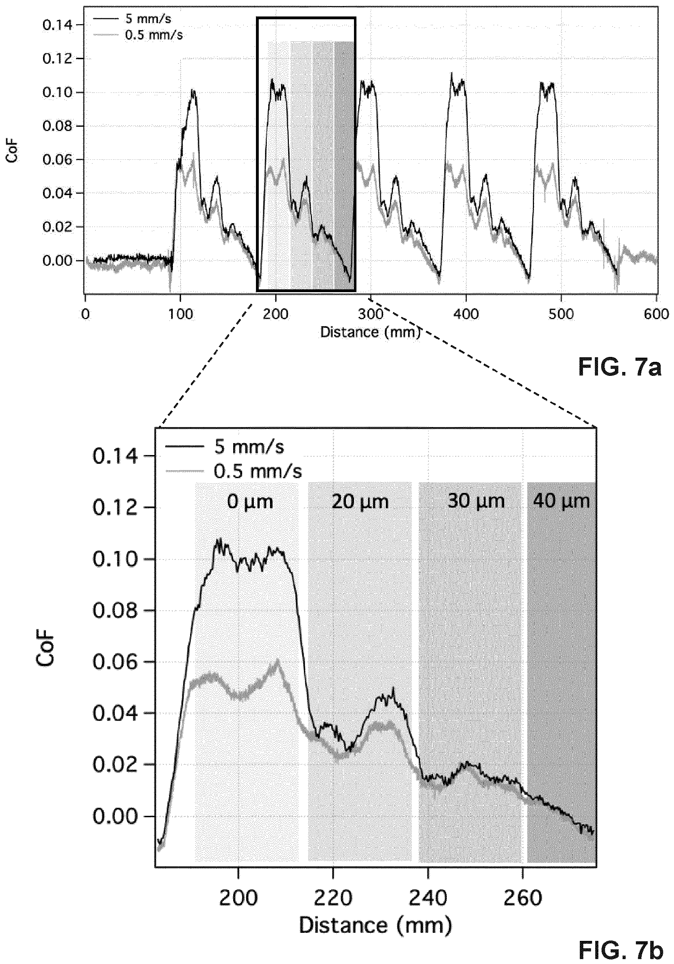

Figures 7a and 7b depict results of gels 3 that were produced from the first reaction mixture 2 described above and that was polymerized as described with reference to figure 4, however this time using a 9-mm-radius glass-molded polyacrylamide spherical pin, leading to a 10 kPa contact pressure at 0.5 N load. The friction measurements were carried out unidirectionally (i.e. circular motion, radius 15 mm), repeatedly passing over the different surface regions that had been polymerized against 0, 20 µm, 30 µm or 40 µm PE. Figures 7a and 7b show the measured coefficient of friction at two different sliding speeds over several full rotations of the gel 3 with differently molded surface regions. The friction results clearly demonstrate the capability of the inventive method for spatially controlling different frictional values over the gel surface by varying the thickness of the source of molecular oxygen 1, in this case of the air-equilibrated PE insert elements 4.

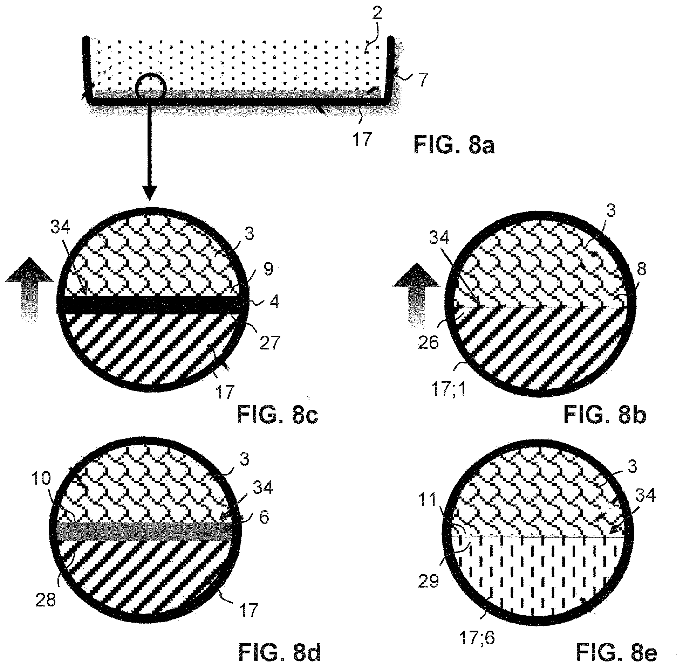

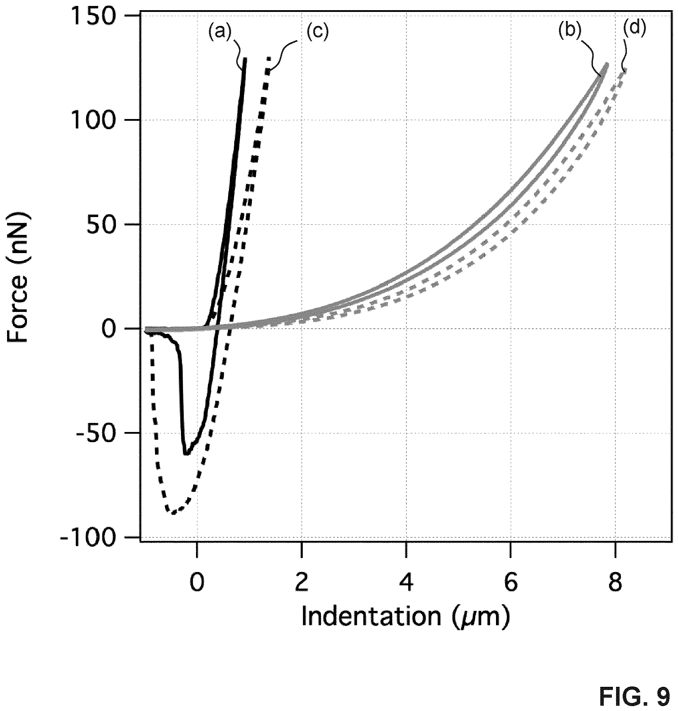

Figures 8a to 8e depicts a series of experiments that were carried out, in which a polymerization solution, i.e. a first reaction mixture 2 as described above, was poured into a vessel 7 as indicated in figure 8a and comprising a bottom surface 17 being provided by an air-equilibrated polystyrene (PS) dish as a source of molecular oxygen 1 in three variants, namely a PS surface without any additional elements (b), an insert element 4 consisting of 10 µm polyethylene foil (oxygen permeable) (c), a blocking element 6 consisting of 10 µm poly(vinylidene dichloride) (PVDC) foil (oxygen impermeable) (d). In figure 8e, a blocking element 6 consisting of a glass dish replaces the PS surface which is used as the control experiment. That is, in said control experiment no source of molecular oxygen was present. UV polymerization was then carried out as described above with reference to figures 2 and 3. As will be illustrated with reference to figure 9, the different surface regions 26-29 of the mold 7 resulted in the formation of different interfaces 34 and ultimately in a gel 3 having different surface regions 8-11.

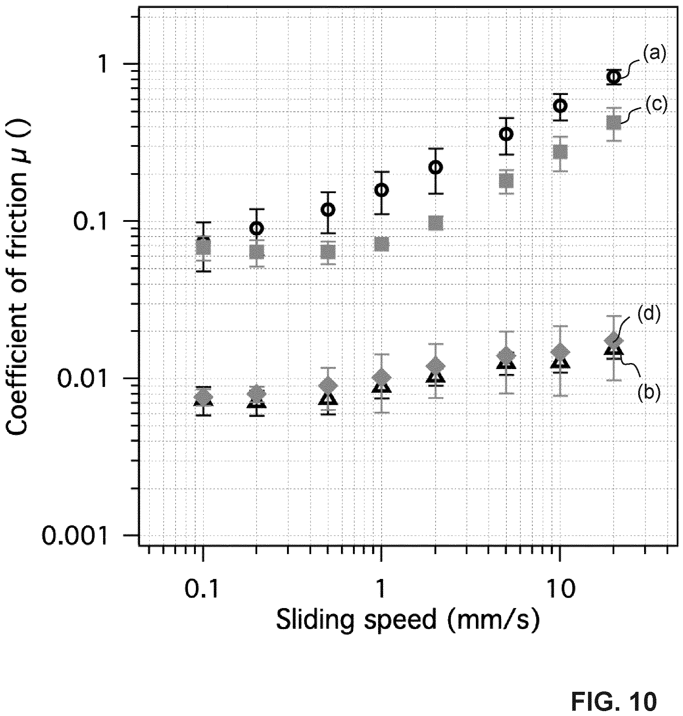

The glass dish 6 is oxygen impermeable, whereas the polystyrene dish is oxygen permeable, and has a high oxygen content after air equilibration. Air-equilibration of the PS dish was carried out by exposing said PS dish to air until an equilibrium oxygen concentration has been attained within the PS. In this regard it should be noted that in principle, most bulk polymers that are used in daily life such as PS, PE, PP, etc., are not just pure polymers, but contain a certain amount of molecular oxygen. If these polymers were in vacuum (or in an oxygen-deprived environment such as pure nitrogen, for example), the oxygen would diffuse out of these polymers, and these polymers would be more or less oxygen-free. Consequently, if these PS molds would be stored in a nitrogen chamber and perform experiments in this chamber, the PS could not serve as an oxygen source, since it would contain none. It is for this reason that the materials used herein as source of molecular oxygen are air-equilibrated or saturated. Figure 9 depicts nanoindentation measurements which were carried as described with reference to figure 2. In fact, and as already well known in the field, the glass-molded surface region of the gel (a) had a high modulus (-30 kPa), while the PS-molded surface of the gel (b) had a much lower elastic modulus (< 1 kPa) and a low friction coefficient, see Figure 10. In fact, friction measurements as depicted in figure 10 and as described with reference to figure 3 showed that the friction coefficient was an order of magnitude higher for the glass-molded surface of the gel compared to the PS-molded surface of the gel. The PE insert element made insignificant difference to the elastic modulus (d) of the resulting gel and its frictional behavior (d), while the oxygen-impermeable (but also hydrophobic) PVDC blocking element led to elastic modulus (c) (and friction behavior (c)) resembling that of glass (a). It therefore appears that PVDC masks the oxygen emanating from the PS and strategic placement of PVDC foil pieces on a PS mold could be used to locally provide areas of relatively high friction on a gel surface.

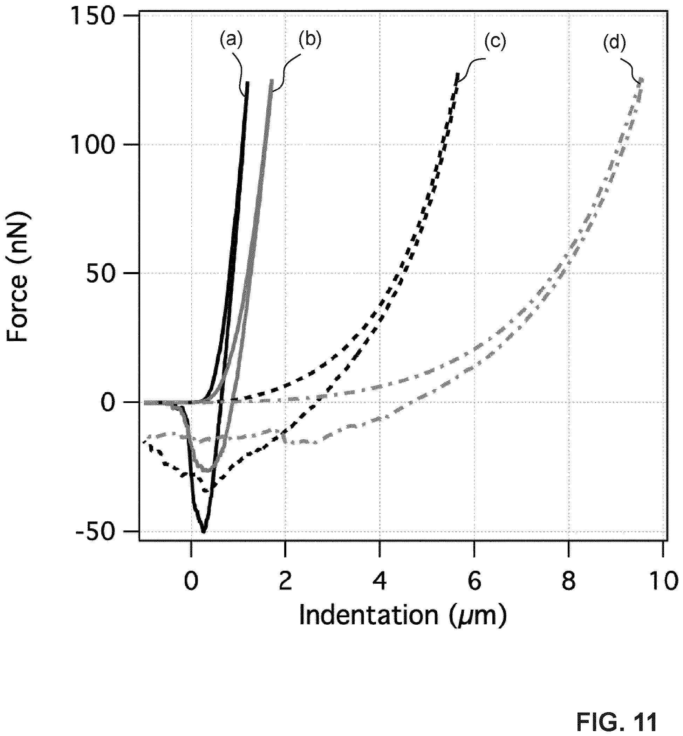

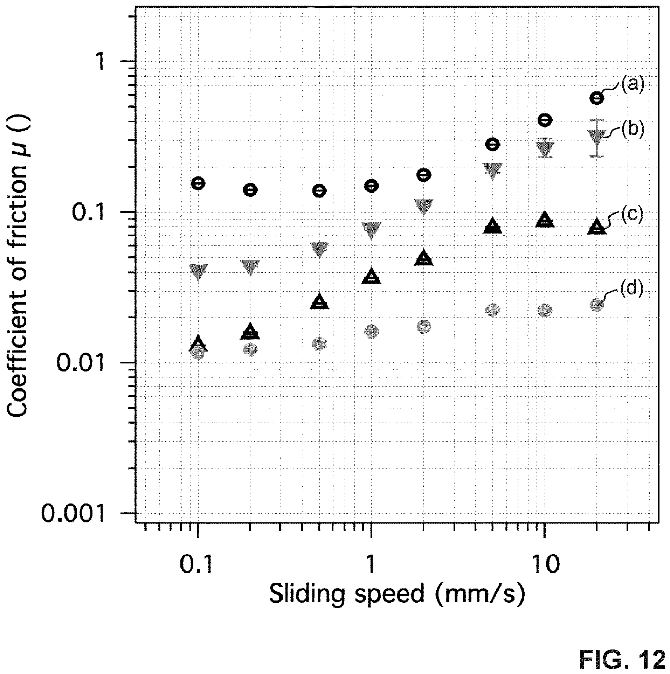

Figures 11 and 12 depict measurement results for gels 3 being produced from a different reaction mixture 2. Namely, this second reaction mixture 2 consisted of a crosslinker-free polymerizing solution, consisting of 10 wt% poly(ethylene glycol) diacrylate (molecular weight 700 Da) (PEGDA) and 0.01 wt% lithium phenyl-2,4,6-trimethylbenzoylphosphinate (LAP). The second reaction mixture 2 was molded in an assembly 12 as disclosed in figures 1a and 1b, and wherein the polymerization reaction corresponded to a UV polymerization as described above with reference to figures 2 and 3.

Indentation measurements (figure 11) and reciprocating friction measurements (figure 12), carried out as described with respect to figures 2 and 3, respectively, show the results for these PEGDA gel surfaces that were synthesized against a separation element 5 in the form of a PE membrane, wherein the source of molecular oxygen 1 was provided as a flowing gas comprising 0.0 % O₂ (a), 0.2 % O₂ (b), 1 % O₂ (c) and 22 % O₂ (d). The results showed that the mechanical and tribological behavior was very similar to that shown for acrylamide. In other words, varying the concentration of molecular oxygen flowing beneath the polyethylene membrane was an effective method of controlling both modulus and friction on the gel surface.

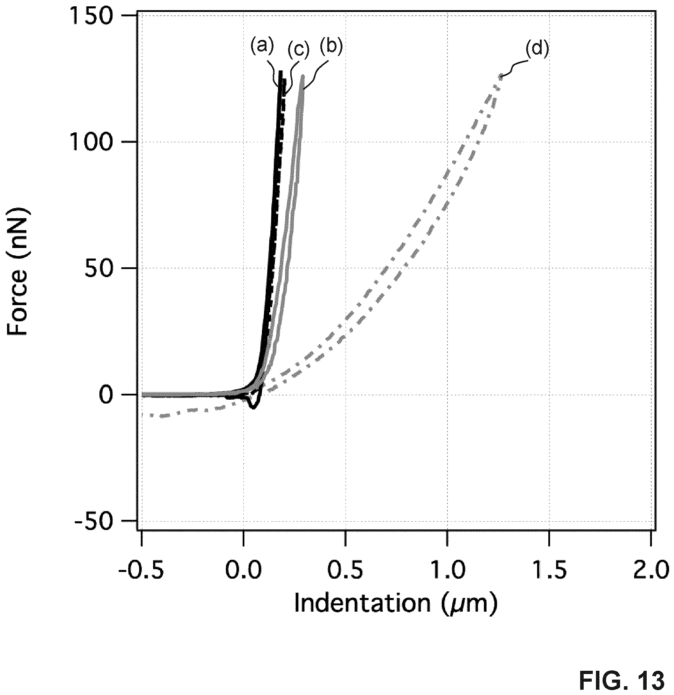

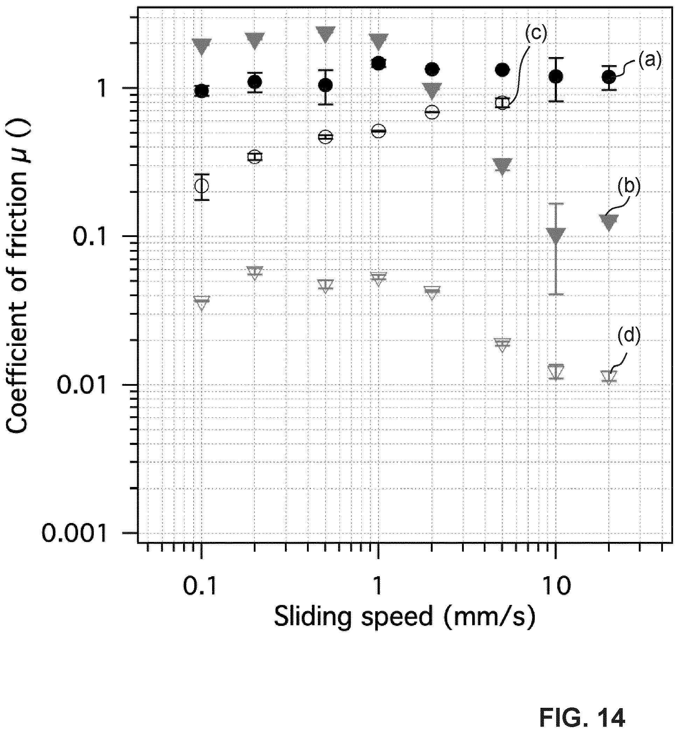

Figures 13 and 14 depict measurement results for gels 3 being produced from a different reaction mixture 2. Namely, this third reaction mixture 2 consisted of a polymerizing solution consisting of 45 wt% hydroxyethyl methacrylate, 15 wt% n-vinyl pyrrolidone, 1.2 wt% ethylene glycol dimethacrylate and 0.1 wt% lithium phenyl-2,4,6-trimethylbenzoylphosphinate (LAP). The polymerization reaction corresponds to a UV polymerization as described above with reference to figures 2 and 3. Indentation measurements (figure 13), carried out as described with respect to figure 2, showed that the surface of the hydrogel 3 is moderately softer (b) (*E* = 190 ± 10 kPa) when air is flowed beneath the separation element 5 in the form of the PE membrane compared to flowing pure nitrogen (a) (*E* = 480 ± 10 kPa). Reciprocating friction measurements (figure 14), carried out as described with respect to figure 3, but with using a 9-mm-radius glass-molded hydroxyethyl-methacrylate spherical pin, leading to a 30 kPa contact pressure at 0.5 N load, showed high coefficient of friction for the gel 3 molded against the PE membrane overflown with pure nitrogen (a). The coefficient of friction for the gel 3 synthesized by flowing air underneath the PE membrane (b) was similarly high at slow sliding speeds, but significantly lower at high sliding speeds, presumably due to the softer surface that facilitated the formation of a hydrodynamic lubricating film. Exposing the synthesized gels 3 to an organic solvent, here to ethanol, resulted in swelling of the gels 3, whereby an organogel was produced and a reduction in the elastic modulus was achieved (see figure 13). In particular, it has turned out in this example that ethanol is a better solvent for the polymer as the polymer swells more and the brushy surfaces are more lubricious as compared to a swelling in water. Compared to the aqueous solvation of the gels according to the previous examples, the surface of the gel 3 synthesized by flowing nitrogen underneath the separation element 5 in the form of the PE membrane had a slightly lower elastic modulus (c) (*E* = 440 ± 30 kPa). The elastic modulus of the PE/air-molded gel (d), however, was significantly lower (*E* = 13 ± 1 kPa) when measured in ethanol compared to water. Performing friction measurements in ethanol showed up to an order of magnitude lower coefficient of friction for the gel 3 synthesized against a PE membrane and air compared to the nitrogen case.

The results indicate that the previously described approaches can be used for controlling the friction of methacrylate-based gels, especially when used in good solvents, as well.

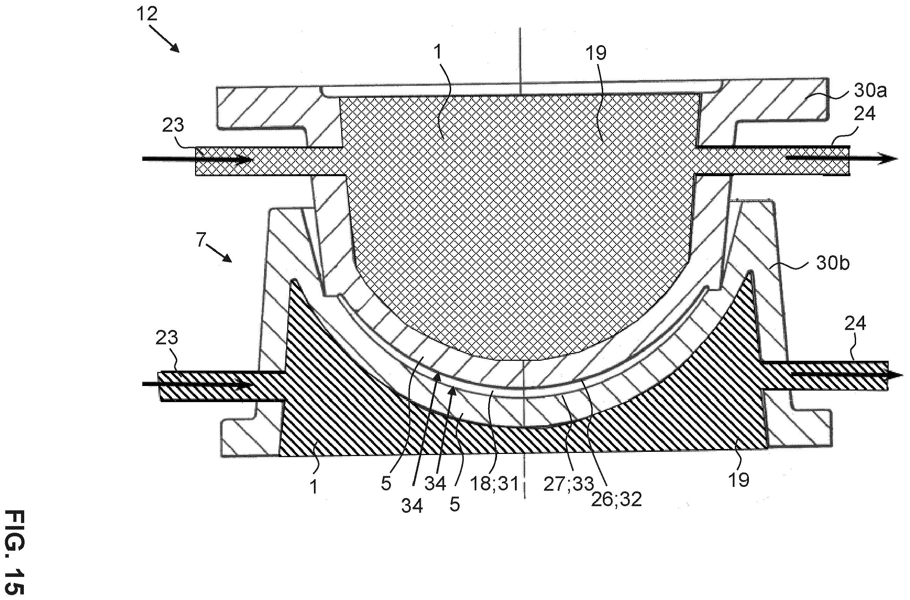

Figures 15 to 17 show other conceivable assemblies 12 for controlling the tribological properties of a gel 3. These assemblies 12 differ from the assemblies 12 shown in Figures 1a, 1b and 4 in the design of the vessel 7 and the source of the molecular oxygen 1. The different designs result from the intended end use of the gel 3. In fact, Figures 15 to 17 show an implementation of the approach according to the invention in molds 7 used for the production of contact lenses. These molds 7 are two-part molds comprising a first mold part 30a and a second mold part 30b. In the assembled state of the mold parts 30a, 30b depicted in figures 15 to 17, the mold parts 30a, 30b enclose an intermediate space 31 in which the reaction mixture 2 is placed to produce the gel 3 for the contact lens. The intermediate space 31 constitutes the reaction chamber 18 as mentioned earlier.

In the embodiment shown in Figure 15, the surface 32, 33 of the mold parts 30a, 30b facing the reaction mixture 2 being comprised in the intermediate space 31 or reaction chamber 18 are in each case formed on an oxygen-permeable separation element 5 that allows diffusion of molecular oxygen from two reservoirs 19 of molecular oxygen. In other words, the two reservoirs 19 comprising the source of molecular oxygen 1 are separated from the reaction chamber 18 and thus from the reaction mixture 2 by the two separation elements 5. In the present examples the source of molecular oxygen 1 is provided by fluids comprising molecular oxygen. Each reservoir 19 is connected to a fluid inlet in the form of a tube 23 and a fluid outlet in the form of another tube 24, respectively, which allow a flow of fluid from the fluid inlet 23 into the reservoir 19 and fluid flow out of the reservoir 19.

The provision of the two separation elements 5 allows the formation of two interfaces 34 between the sources of molecular oxygen 1 and the reaction mixture 2. Further, this arrangement allows the polymerization of the reaction mixture 2 to result in a gel 3 whose first surface facing the first surface 32 of the mold part 30a and thus the first separation element 5 has a different tribological property than a second surface of the gel facing the second surface 33 of the second mold part 30b and thus the second separation element 5. This is achieved here by the first fluid in the first reservoir 19 being in connection with the first mold part 30a having a different concentration of molecular oxygen than a second fluid in the second reservoir 19 being in connection with the second mold part 30b. Put differently, the inventive approach can be used in contact-lens manufacture, providing the capability of imparting different friction coefficients to different sides of the lens, by controlling the oxygen content of the fluid inputs in a mold 7, in which the molding walls 32, 33 are oxygen permeable and thin.

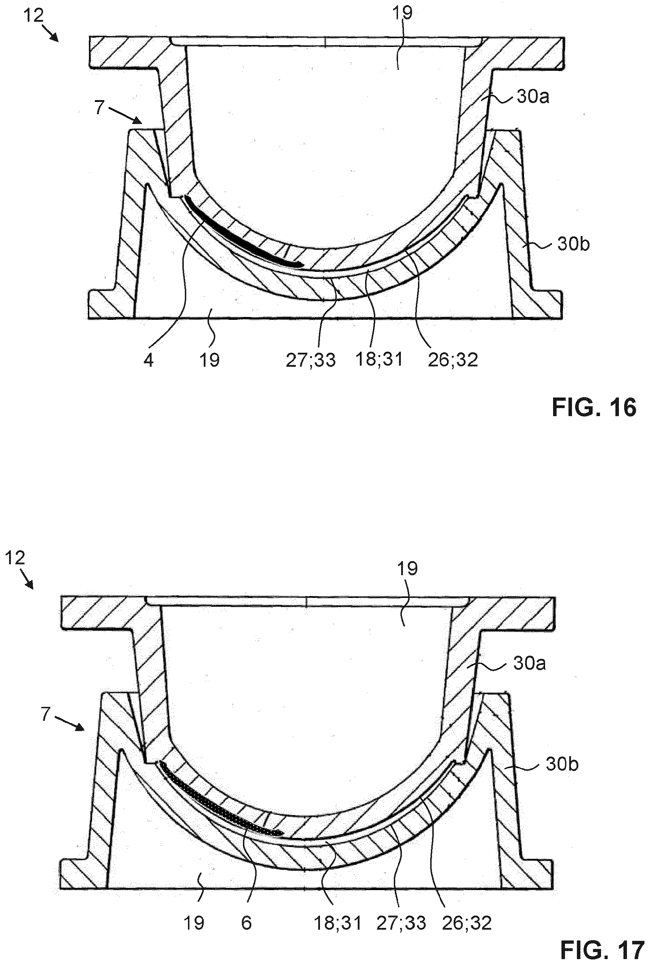

Figure 16 depicts a controlling of the tribological properties of a gel 3 which is achieved by the provision of an oxygen-releasing insert element 4 that is arranged on the surface 32 of the first mold part 30a facing the intermediate space 31. Here, said insert element 4 is provided by a polyethylene foil or membrane that is arranged on a surface region of the oxygen-impermeable wall of the first mold part 30a so as to provide a locally lubricious region on the surface region of the gel 3 facing said insert element 4.

Figure 17 depicts a controlling of the tribological properties of a gel 3 which is achieved by the provision of oxygen-saturated molding walls one of which is patterned with a blocking element 6 that is oxygen-impermeable, such as a poly(vinylidene dichloride) foil. The blocking element 6 provides a locally high-friction region, which can be used to inhibit lens rotation in the eye, for example.

Hence, from the above it becomes apparent that the present invention allows the controlling of tribological properties such as the coefficients of friction and their spatial distribution over the surfaces of a gel. To this end the polymerization of a reaction mixture is taking place such that the surface of interest of the gel is formed against a vessel or mold through which or from which molecular oxygen is diffused in a controlled way. The presence and the amount of oxygen being released from the vessel or molding surface controls the degree of crosslinking in the gel surface, which in turn determines its tribological properties.

**LIST OF REFERENCE SIGNS**

| | | | |
|---|---|---|---|
| 1 | source of molecular oxygen | 26 | surface region |
| 2 | reaction mixture | 27 | surface region |
| 3 | gel | 28 | surface region |
| 4 | insert element | 29 | surface region |
| 5 | separation element | 30a | mold part |
| 6 | blocking element | 30b | mold part |
| 7 | vessel | 31 | intermediate space |
| 8 | surface region | 32 | surface |
| 9 | surface region | 33 | surface |
| 10 | surface region | 34 | interface |
| 11 | surface region | | |
| 12 | assembly | | |
| 13 | side wall | F | flow direction |
| 14 | side wall | WV | width vessel |
| 15 | side wall | LV | length vessel |
| 16 | side wall | HV | height vessel |
| 17 | bottom wall | LS | length separation element |
| 18 | reaction chamber | WS | width separation element |
| 19 | reservoir | HS | height separation element |
| 20 | upper side | TS | thickness separation element |
| 21 | side wall | HI | height insert element |
| 22 | side wall | TI | thickness insert element |
| 23 | tube | LI | length insert element |
| 24 | tube | HR | height reservoir |
| 25 | upper side | | |

## Claims

1. A method of controlling at least one tribological property of a gel (3) comprising one or more crosslinked polymers, the method comprising the steps of:
- Providing at least one source of molecular oxygen (1) that is interfaced with a reaction mixture (2); and
- Producing the gel (3) from the reaction mixture (2) while molecular oxygen from the source of molecular oxygen (1) is present at least at an interface (34) between the reaction mixture (2) and the source of molecular oxygen (1) during at least one period in time.

2. The method according to claim 1, wherein the molecular oxygen being present at the interface (34) is kept constant during the at least one period in time or is varied during the at least one period in time, and/or
wherein the molecular oxygen is present at the interface (34) during an entire period of production of the gel (3) or during a part of the entire period of production of the gel (3), and/or
wherein a concentration of the molecular oxygen at the interface (34) and/or in the source of molecular oxygen (1) is used to control the tribological property of the gel (3), and/or
wherein a release rate associated with the release of molecular oxygen from the source of molecular oxygen (1) into the reaction mixture (2) is used to control the tribological property of the gel (3).

3. The method according to any one of the preceding claims, wherein the source of molecular oxygen (1) is provided by at least one of:
- one or more fluids comprising or consisting of molecular oxygen, and/or
- one or more chemical reactions producing molecular oxygen, and/or
- one or more electrolytic systems producing molecular oxygen, and/or
- one or more compounds (4) comprising molecular oxygen and being configured to release the molecular oxygen, and/or
wherein the source of molecular oxygen (1) is arranged separately from the reaction mixture (2) or wherein the source of molecular oxygen (1) is arranged at least partially in contact with the reaction mixture (2).

4. The method according to any one of the preceding claims, further comprising the step of providing at least one separation element (5) that separates the source of molecular oxygen (1) from the reaction mixture (2), and wherein the separation element (5) preferably is at least partially oxygen-permeable.

5. The method according to claim 4, wherein the separation element (5) corresponds to a layer or membrane, and/or
wherein the separation element (5) has an oxygen permeability of at least 10 cm³·25µm/(m²·24h·atm), preferably of at least 100 cm³·25µm/(m²·24h·atm), more preferably of at least 1000 cm³·25µm/(m²·24h·atm), and/or in the range of 10 cm³·25µm/(m²·24h·atm) to 10'000 cm³·25µm/(m²·24h·atm),
wherein the separation element (5) comprises or consists of one or more polymers or copolymers thereof such as polyethylene and/or polystyrene and/or polytetrafluoroethylene and/or polypropylene and/or polyvinyl chloride, and/or
wherein a thickness of the separation element (5) is constant over a length of the separation element (5) or varies along the length of the separation element (5) and preferably is at least 1 nanometer or more, preferably at least 100 nanometer or more, and/or in the range of 1 nanometer to 1000 micrometer.

6. The method according to any one of the preceding claims, wherein the source of molecular oxygen (1) is provided by at least one insert element (4) that is at least partially in contact with the reaction mixture (2), and
wherein the insert element (4) comprises or consists of one or more compounds comprising molecular oxygen and being configured to release molecular oxygen, and/or
wherein the insert element (4) is oxygen-saturated.

7. The method according to claim 6, wherein the insert element (4) corresponds to a layer or a membrane, and/or
wherein the insert element (4) comprises or consists of one or more polymers or copolymers thereof such as polyethylene and/or polystyrene and/or polytetrafluoroethylene and/or polypropylene and/or polyvinyl chloride, and/or
wherein a thickness of the insert element (4) is constant over a length of the insert element (4) or varies along the length of the insert element (4) and preferably is at least 1 nanometer or more, preferably at least 100 nanometer or more, and/or in the range of 1 nanometer to 1000 micrometer.

8. The method according to any one of the preceding claims, further comprising the step of at least partially blocking a supply of molecular oxygen into the reaction mixture (2), preferably by providing at least one blocking element (6) particularly preferably at the interface (34) between the reaction mixture (2) and the source of molecular oxygen (1).

9. The method according to any one of the preceding claims, further comprising the steps of providing the reaction mixture (2) in a mold (7) and producing the gel (3) by molding the reaction mixture (2) while molecular oxygen is present at least at an interface (34) between the mold (7) and the reaction mixture (2).

10. Use of the method according to any one of the preceding claims for the manufacturing of a medical device, the medical device preferably being an ophthalmological device such as a contact lens and/or a catheter, and/or
for the manufacturing of a device being suitable for applications on and/or in the human or animal body, the device preferably being a condom.

11. A gel (3) comprising one or more crosslinked polymers, preferably produced by the method according to any one of claims 1 to 9,
wherein the gel (3) comprises at least a first surface region (8) and a second surface region (9),
wherein the first surface region (8) is associated with at least one first tribological property and the second surface region (9) is associated with at least one second tribological property, and
wherein the first tribological property and the second tribological property are different from one another.

12. The gel (3) according to claim 11, wherein a difference between the first tribological property and the second tribological property is at least one order of magnitude, and/or
wherein the first surface region (8) and the second surface region (9) are arranged adjacent to one another or at a distance from one another, and/or
further comprising a pattern that comprises at least one or more first surface regions (8) and one or more second surface regions (9), and/or
wherein at least one or more first surface regions (8) and one or more second surface regions (9) are arranged such that their associated first and second tribological properties exhibit a gradient.

13. A vessel (7) for controlling at least one tribological property of a gel (3) comprising one or more crosslinked polymers, wherein the vessel (7) is configured to receive a reaction mixture (2),
wherein the vessel (7) is configured to be interfaced with at least one source of molecular oxygen (1), and
wherein the vessel (7) is further configured such, that the gel (3) is produced from the reaction mixture (2) while molecular oxygen from the source of molecular oxygen (1) is present at least at an interface (34) between the reaction mixture (2) and the source of molecular oxygen (1) during at least one period in time.

14. An assembly (12) for controlling at least one tribological property of a gel (3) comprising one or more crosslinked polymers, the assembly (12) comprising:
- at least one vessel (7), in particular a mold, that is configured to receive a reaction mixture (2); and
- at least one source of molecular oxygen (1) that is interfaced with the at least one vessel (7),
wherein the assembly (12) is configured such, that the gel (3) is produced from the reaction mixture (2) while molecular oxygen from the source of molecular oxygen (1) is present at least at an interface (34) between the reaction mixture (2) and the source of molecular oxygen (1) during at least one period in time.

15. Use of a source of molecular oxygen (1) for controlling at least one tribological property of a gel (3) comprising one or more crosslinked polymers.
